# EUROPEAN PATENT APPLICATION

(11) **EP 1 586 896 A1**
(43) Date of publication of application: **19.10.2005**
(21) Application number: 04076149.6
(22) Date of filing: 14.04.2004
(51) Int. Cl.: G01N 33/02

(54) **Method and means for quality control of perishable goods**

(71) Applicant: Nederlandse Organisatie voor Toegepast-Natuurwetenschappelijk Onderzoek TNO, 2628 VK Delft (NL)
(72) Inventor: van den Broek, Augusta, Wilhelmina, Johanna Coloma, 5644 BD Eindhoven (NL); Aartsen, Hermanus, Josephus, 5524 AN Steensel (NL); van Zanten, Albertus, 5235 DP's-Hertogenbosch (NL); Doodeman, Gerardus, Johannes, Nicolaas, 5501 DM Veldhoven (NL); Ehlhardt, Huub, 5223 BB Den Bosch (NL); van den Heuvel, Luc A., 2715 AG Zoetermeer (NL); Kruf, Waltherus, Petrus, Maria, 4661 JZ Halsteren (NL)
(74) Representative: Winckels, Johannes Hubertus F.

(57) **Abstract**

Method and means for detecting the quality of a mass. One or more sensors (1) may be placed within or in the vicinity of the mass at a relevant location. Each sensor comprises a remotely readable electrical property, e.g. the RF resonance frequency and/or RF impedance, which is a function of the quality ("fresh"/"contaminated") of the mass. Means (9) are provided for remotely reading the value of the electrical property of the sensor. At a first stage in time and/or place, at which the mass has a known ("good") quality, the measured value is set as a nominal or reference value. At a second stage in time and/or place, at which the mass has an unknown quality, the value is also measured and compared with the reference value. An alarm may be given when the difference between both values passes a certain threshold value.

## Description

### FIELD OF THE INVENTION

The invention refers to quality control of mass goods like food or non-food pastas, slurries, liquids and the like which may susceptible for degradation, contamination etc.

### BACKGROUND OF THE INVENTION

Several methods and systems are known in which the relevant mass is fed along some kind of a detection "port" that e.g. measures the electric property of the mass, flowing along or through the detection port. However useful for the quality control of unpacked mass flows etc., those known methods and systems are not or less fit for quality control of the contents of e.g. bottled or otherwise packed mass substances.

For quality control of bottled or packed masses, systems are known in which use is made of e.g. markers which discolour under the influence of e.g. bacteria etc.

### SUMMARY OF THE INVENTION

The present invention aims to offer an advanced method and means for quality control of masses e.g. packed in glass, plastic or (coated) paper packages.

One aspect of the new method and system is to make use of sensors that may reside within the relevant package, which sensors will be influenced by the quality of the mass inside the package.

The present method in general comprises the following steps:
- have one or more sensors, each being placed within or in the vicinity of the mass at a relevant location, each sensor having a remotely readable electrical property which is a function of the quality of the mass (viz. the mass in the vicinity of the sensor, the "local mass");
- remotely read the value of said electrical property of the relevant sensor.

The (local) mass "under test" has to influence one or more electrical properties of the sensor. The electrical properties of the sensors (e.g. its RF resonance frequency and/or RF impedance) may be influenced by the properties of the mass inside the package: for instance the RF resonance frequency and/or the RF impedance may be different when the mass inside the package is fresh or is contaminated.

The sensor's electrical property preferably may be read-out remotely: wireless and/or from a certain distance.

The method preferably may comprise a step in which the value of the sensor's electrical property is compared with a reference value. To that end e.g. the following steps may be performed:
- read at a first stage in time and/or place, at which first stage the local mass has a known quality ―e.g. fresh (food)―, the value of the electrical property of the relevant sensor;
- read at a second stage in time and/or place, at which second stage the local mass has an unknown quality ―e.g. fresh or contaminated-, the value of the electrical property of the relevant sensor;
- compare both values.

The first step may be used to create a reference (or nominal) value for the sensor's electrical property corresponding with the mass's nominal ("fresh") state, to which the value read in the second step is referenced or compared in the third step: a difference between the reference value and the actual value may indicate degradation of the quality of the mass within the package. It is noted that the reference value may be recorded at a first stage in time and/or place, while the actual value may be recorded at a second stage in time and/or place. Separate stages in time may indicate two different moments at which the reference and actual electrical values are recorded, e.g. the moment at which the relevant package was filled with (fresh) mass, and a moment ―later in time- at which a need was felt to control the quality (freshness) of the same packaged mass. Separate stages in place may indicate two different places (e.g. at the same moment) where the reference and actual electrical values are recorded, e.g. at different production locations or lines e.g. for sake of mutual comparison of the production processes.

An alarm may be given when the actual value read passes a certain threshold value. When, optionally, two values ―gathered at different moments and/or locations, are mutually compared, an alarm may be given when the difference between both values passes a certain threshold value (which threshold may or may not be equal to zero).

The remotely readable electrical properties may comprise the sensor's electrical resonance frequency and/or the sensor's electrical impedance.

### FIGURES

Figures 1a to 1c show some exemplary embodiments of a sensor to be used in a system fit for performing the method presented above.
Figures 2a and 2b show an exemplary embodiment of a system which is fit for performing the method presented above.

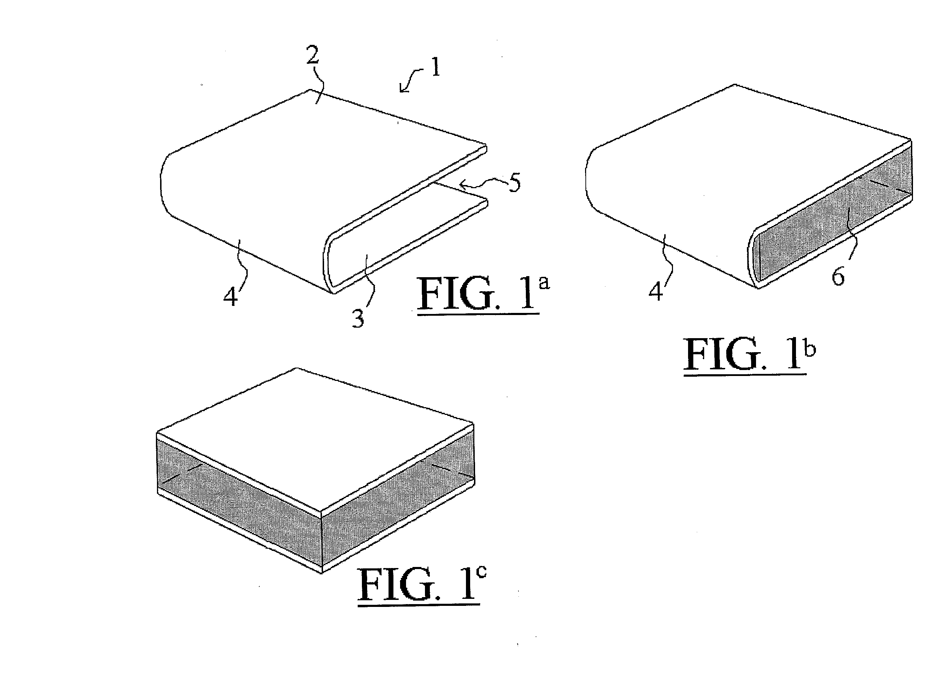

Figure 1a shows a first exemplary embodiment of a sensor 1 that is fit to be used in a system fit for performing the method presented above. The sensor may comprise electrically conductive plates 2 and 3, interconnected by a connection "strip" 4. The plates 2 and 3 and the strip 4 together form a resonator, having its resonance frequency within the RF (radio frequent) domain. The construction and operation of sensor 1 is based on that of a microstrip patch antenna, which in fact is a capacitor having two plates. If connected by strip 4 one of the dimensions of the individual plates are about a quarter of the operation wave length. When not interconnected, one of the plate dimensions are about half the operation wave length. The plates 2 and/or 3 may be perforated by one or more holes. Besides the plate dimensions, the resonance frequency and/or impedance of the "microstrip" sensor 1 will be influenced by the characteristics of the dielectricum between the plates 2 and 3.

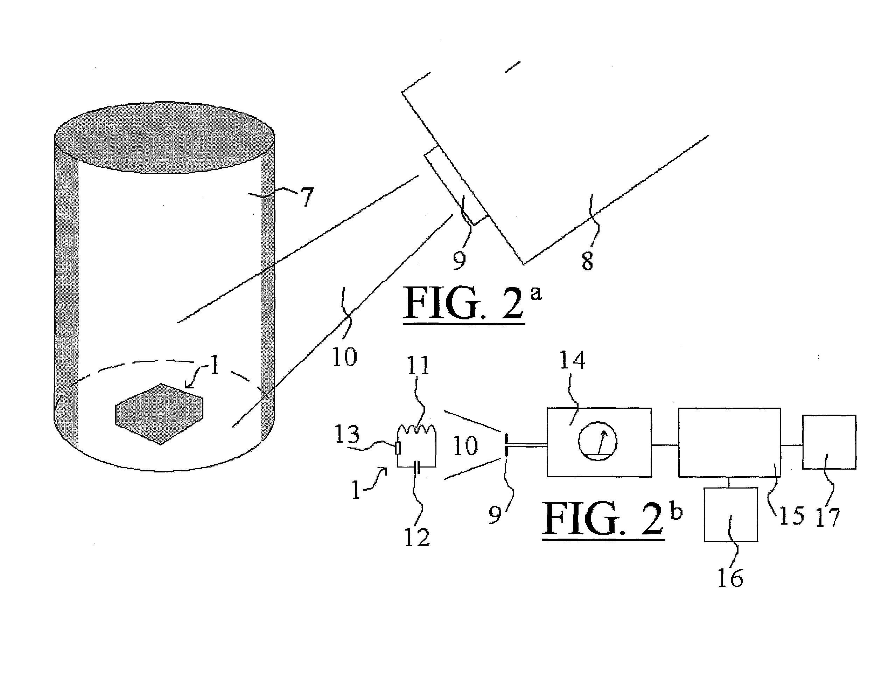

In the embodiment of figure 1a the gap 5 between the resonator's parts 2 and 3 is not filled; in use within a mass (see figure 2a) to be monitored the gap 5 may be filled with the mass. Optionally, the gap 5 may be filled by a solid dielectricum 6, which may or may not be partially filled or otherwise influenced by the (e.g. fluid) mass to be monitored; in that case the strip 4 might be unnecessary, as is shown in figure 1c. The dielectricum 6 may be fit to adsorb or absorb part of the mass to be monitored.

The sensor 1 is fit to be placed within or in the vicinity of the mass to be monitored at a relevant location. The sensor 1 has an electrical property, viz. e.g. its RF resonance frequency and/or impedance, which is a function of the quality of the local mass, viz. due to the influence of the mass quality to the dielectric properties in and around the sensor's gap 5, filled or not filled with a solid dielectricum 6. The sensor's RF resonance frequency and/or impedance are remotely readable by remote reading means as will be discussed below.

Figures 2a and 2b show an exemplary embodiment of a system using sensors as discussed above. The system shown in figures 2a and 2b is fit for detecting the quality of a mass e.g. pasteurised milk or milk powder, packed in a package 7. The system comprises means fit for remotely reading the value of an electrical property, e.g. the RF resonance frequency and/or impedance of "microstrip" sensors 1, each to be placed within the mass to be monitored. In fact the sensor's relevant value is influenced by a "local mass", viz. the mass in the direct vicinity of the sensor 1 (e.g. by adsorption or absorption part of that mass) and thus the sensor's -remotely readable- RF electrical property is a function of the quality of the mass, which is considered to be representative for the mass is the whole package 7. The sensor's RF property ―its resonance frequency and/or impedance- is read by means of an RF scanner 8, able to transmit and receive an RF signal via an antenna 9. For instance an RF signal is transmitted having the resonance frequency of the sensor 1 in when the mass ―which influences the sensor's resonance frequency and/or the RF impedance- has its "normal" constitution ("fresh"). At that resonance frequency the sensor's impedance will be e.g. minimal (or maximal, depending on the sensor's exact constitution). When, however, the constitution of the mass is not "fresh" but degraded ("contaminated"), the sensor's resonance frequency will be changed due to a change of the sensor's dielectricum, at least partly formed by the dielectric behaviour of the local mass, which is different in the "fresh" and "contaminated" state respectively. Consequently, the impedance for the RF signal transmitted by the scanner will be changed. Measuring the state of the mass within the package 7 may be performed by either measuring the actual transmission power, e.g. the antenna current, at a fixed transmission frequency or measuring the actual resonance frequency of the sensor 1, e.g. by varying the scanner's transmission frequency and detecting the frequency at which the transmission power (e.g. antenna current) is minimal (or maximal respectively). Using either method, degradation of the package's content will result in a changed RF property, viz. the sensor's resonance frequency or its impedance for a fixed frequency, which change is detectable by the scanner 8.

To be able to detect which RF value is "good" and which is "bad" the system preferably comprises means for reading (measuring) at a (first) stage in time and/or place at which the mass has a known ("good") quality, the value of the relevant electrical property of the sensor which value consequently is considered to be the reference ("good") value. The system, moreover, comprises means for reading (measuring) at a (second) stage in time and/or place at which the mass has an unknown ("good" or "bad") quality, the (actual) value of the electrical property of the sensor, and, finally, means for comparing the actual value with the reference value.

Turning now to figure 2b, showing a schematic diagram of the scanner 7, the scanner 7 may comprise, besides the antenna 9, transmitting and receiving an RF signal 10, a transceiver unit 14, a processor 15, a memory 16 and an output unit 17. At a first stage in time (e.g. a first ―trusted- moment in the mass's production process) and/or place (e.g. a first ―trusted- production location), at which the local contents of the package 7 in the neighbourhood of the sensor 1 (presented here as a simple RF resonance circuit 11-12-13) has a known ("good") quality, the RF value (e.g. impedance) is measured. The "good quality" RF value may be stored in the memory 16 as reference value. At a second stage in time and/or place (e.g. another production line, to be monitored), at which the local contents of the package 7 in the neighbourhood of the sensor 1 has an unknown quality, the RF value (e.g. impedance) is measured. Both measurements -at the first and the second stage- may be performed by the same scanner 8 or by different copies of the scanner 8 which may be coupled to be able to exchange their measured values, especially the relevant reference value, corresponding with the mass qualification "good", stored in the memory 16 of the relevant scanner 8. The processor 15 of the relevant scanner 8 is fit for comparing both values measured in the first and in the second stage, the reference value, stored in the memory 16 and the measured actual RF value. The processor 16 may comprise means for alarming when the actual value read passes a certain threshold value, or when the difference between the reference value and the actual value passes a certain threshold value, indicating that the quality of the monitored contents of the package 7 passes a certain quality level in comparison with the nominal quality level indicated by the reference RF value, the sensor's nominal resonance frequency and/or its electrical impedance for RF, detected in the first measuring stage and stored in the memory 16.

## Claims

1. Method for detecting the quality of a mass, comprising steps of:
- have one or more sensors, each being placed within or in the vicinity of the mass at a relevant location, each sensor having a remotely readable electrical property which is a function of the quality of the mass;
- remotely read the value of said electrical property of the relevant sensor.

2. Method according to claim 1, comprising a step in which the value of the sensor's electrical property is compared with a reference value.

3. Method according to claim 2, comprising steps of:
- read at a first stage in time and/or place, at which first stage the mass has a known quality, the value of the electrical property of the relevant sensor;
- read at a second stage in time and/or place, at which second stage the mass has an unknown quality, the value of the electrical property of the relevant sensor;
- compare both values.

4. Method according to claim 1, an alarm being given when the value read passes a certain threshold value.

5. Method according to claim 2, an alarm being given when the difference between both values passes a certain threshold value.

6. Method according to claim 1, said remotely readable electrical property comprising the relevant sensor's electrical resonance frequency.

7. Method according to claim 1, said remotely readable electrical property comprising the relevant sensor's electrical impedance.

8. System for detecting the quality of a mass, the system comprising means fit for remotely reading the value of an electrical property of one or more sensors (1), each to be placed within or in the vicinity of said mass at a relevant location, the remotely readable electrical property being a function of the quality of the mass.

9. System according to claim 8, comprising means for comparing the value of the sensor's electrical property with a reference value.

10. System according to claim 9, comprising means (8) for reading at a first stage in time and/or place, at which first stage the mass has a known quality, the value of said electrical property of the relevant sensor, means (8) for reading at a second stage in time and/or place, at which second stage the mass has an unknown quality, the value of the electrical property of the relevant sensor, and means (15,16) for comparing both values.

11. System according to claim 8, comprising means (17) for alarming when the value read passes a certain threshold value.

12. System according to claim 9, comprising means (17) for alarming when the difference between both values passes a certain threshold value.

13. System according to claim 8, the sensor's remotely readable electrical property comprises its electrical resonance frequency.

14. System according to claim 8, the sensor's remotely readable electrical property comprises its electrical impedance.

15. Sensor, fit to be used in a system for detecting the quality of a mass, the sensor being fit to be placed within or in the vicinity of the mass at a relevant location, the sensor, moreover, having an electrical property which is a function of the quality of the mass and which is remotely readable by remote reading means (8), fit for remotely reading the value of said electrical property.

16. Sensor according to claim 15, the sensor's remotely readable electrical property comprises its resonance frequency.

17. Sensor according to claim 15, the sensor's remotely readable electrical property comprises its impedance.
